# EUROPÄISCHE PATENTSCHRIFT

(11) **EP 0 630 487 B1**
(45) Veröffentlichungstag und Bekanntmachung des Hinweises auf die Patenterteilung: **27.11.1996**
(21) Anmeldenummer: 93905267.6
(22) Anmeldetag: 20.02.1993
(51) Int. Cl.: G02B 23/24

(54) **VERFAHREN UND VORRICHTUNG ZUR DARSTELLUNG EINES ARBEITSBEREICHES IN EINER DREIDIMENSIONALEN STRUKTUR**
PROCESS AND DEVICE FOR REPRESENTING A WORK AREA IN A THREEDIMENSIONAL STRUCTURE
PROCEDE ET DISPOSIF DE REPRESENTATION D'UNE ZONE DE TRAVAIL D'UNE STRUCTURE EN TROIS DIMENSIONS

(30) Priorität: 12.03.1992 DE 4207901
(43) Veröffentlichungstag der Anmeldung: 28.12.1994
(73) Patentinhaber: AESCULAP AG, D-78501 Tuttlingen (DE)
(72) Erfinder: SCHULZ, Hans-Joachim, D-7200 Tuttlingen (DE); TÜMMLER, Hanns-Peter, D-7200 Tuttlingen (DE); WIENEKE, Paul, D-7200 Tuttlingen (DE)
(74) Vertreter: Hoeger, Stellrecht & Partner
(86) Internationale Anmeldenummer: EP9300409
(87) Internationale Veröffentlichungsnummer: WO9318426

(56) Entgegenhaltungen:
- EP-A- 0 352 952
- DE-A- 4 038 125
- PATENT ABSTRACTS OF JAPAN vol. 13, no. 546 (C-661) 6. Dezember 1989 & JP-A-12 23 926

## Beschreibung

Die Erfindung betrifft ein Verfahren zur Darstellung eines Arbeitsbereiches in einer dreidimensionalen Struktur, bei dem man den Arbeitsbereich mit einem Betrachtungsgerät abbildet, die Positionierung des Betrachtungsgeräts relativ zu der Struktur bestimmt, die Koordinaten eines Verschiebewegs für ein Instrument oder für das Betrachtungsgerät in der Struktur vorbestimmt und sie mit der jeweiligen Positionierung vergleicht.

Die Erfindung betrifft weiterhin eine Vorrichtung zur Darstellung eines Arbeitsbereiches in einer dreidimensionalen Struktur.

Aus der JP-A-1 223 926 ist eine Vorrichtung zur Betrachtung eines Arbeitsbereiches bekannt, bei der auf einer Darstellungseinheit das tatsächliche Bild überlagert wird vom Verschiebeweg eines Endoskops.

Bei der Betrachtung von dreidimensionalen Strukturen mit Betrachtungsgeräten, also beispielsweise mit Endoskopen oder Mikroskopen, wird von den Betrachtungsgeräten eine senkrecht auf der optischen Achse des Betrachtungsgerätes stehende, in der Brennebene des Betrachtungsgerätes angeordnete Ebene scharf abgebildet, so daß ein Betrachter entweder unmittelbar oder unter Zwischenschaltung einer Kamera und eines Monitors mittelbar eine Ebene der dreidimensionalen Struktur betrachten kann. Die dreidimensionale Struktur kann dabei beliebig sein, es kann sich beispielsweise um das Innere einer Maschine, eines biologischen Präparates oder eines menschlichen oder tierischen Körpers handeln. Diese Strukturen sind oft außerordentlich kompliziert, so daß es günstig ist, nicht erst bei der eigentlichen Betrachtung der Struktur zu entscheiden, wie das Betrachtungsgerät oder ein Instrument in der Struktur vorgeschoben werden soll, um eine bestimmte Stelle zu erreichen, sondern es erweist sich als günstig, den Vorschubweg vorher zu planen.

Es ist Aufgabe der Erfindung, ein Verfahren der gattungsgemäßen Art so auszugestalten, daß bei der Betrachtung einer dreidimensionalen Struktur der vorgeplante Verschiebeweg für das Betrachtüngsgerät oder für ein Instrument für den Betrachter unmittelbar sichtbar wird, so daß er bei der Betrachtung der Struktur unmittelbar die Vorschiebebewegung des Betrachtungsgerätes oder eines Instrumentes steuern kann.

Diese Aufgabe wird bei einem Verfahren der eingangs beschriebenen Art erfindungsgemäß dadurch gelöst, daß man in der Abbildung des Arbeitsbereichs den vorbestimmten Verschiebeweg ortsrichtig als Durchstoßpunkt des Verschiebewegs durch die jeweils beobachtete Ebene der Struktur darstellt.

Durch eine genaue Messung der relativen Positionierung des Betrachtungsgerätes relativ zu der Struktur kann man genau bestimmen, welche Ebene in der Struktur im Betrachtungsgerät abgebildet wird, nämlich eine senkrecht durch die optische Achse des Betrachtungsgerätes verlaufende und in der Brennebene des Betrachtungsgerätes liegende Ebene. Für jede beliebige Relativposition des Betrachtungsgerätes relativ zu der Struktur wird also eine andere Ebene scharf abgebildet.

Nach der Bestimmung dieser Ebene kann man den Schnittpunkt dieser Ebene mit einem vorbestimmten Verschiebeweg in der Struktur berechnen und dadurch genau bestimmen, in welcher Position der abgebildeten Ebene der vorbestimmte Verschiebeweg diese Ebene durchstößt. Wenn die optische Achse des Betrachtungsgerätes genau im Durchstoßpunkt des vorbestimmten Verschiebeweges angeordnet ist, befindet sich dieser Durchstoßpunkt in der Mitte der beobachteten Fläche, bei einer seitlichen Abweichung des Verschiebeweges von der optischen Achse im Abstand zum Mittelpunkt der Abbildung. Es ist üblich, bei der Beobachtung der Ebenen die Position der optischen Achse beispielsweise durch ein Fadenkreuz anzudeuten, so daß mit anderen Worten aufgrund der Bestimmung der Lage der beobachteten Ebene und des Vergleiches der Koordinaten dieser Ebene mit den Koordinaten des vorbestimmten Verschiebeweges der Abstand und die Richtung bestimmt werden können, die von dem Fadenkreuz aus zurückgelegt werden müssen, um zum Durchstoßpunkt des Verschiebeweges in der beobachteten Ebene zu gelangen.

Dieser Durchstoßpunkt in der beobachteten Ebene wird bei dem erfindungsgemäßen Verfahren zusätzlich dargestellt, beispielsweise durch Überlagerung auf einem Monitor oder durch Einblenden eines entsprechend positionierten Bildes in den Strahlengang des Mikroskopes. Der Beobachter kann also gleichzeitig das tatsächlich durch das Betrachtungsgerät übermittelte Bild der abgebildeten Ebene beobachten und eine Markierung, die den Durchstoßpunkt des vorbestimmten Verschiebeweges durch diese Ebene anzeigt.

Wenn der Verschiebeweg für die Verschiebebewegung des Betrachtungsgerätes selbst bestimmt ist, kann durch seitliche Verschiebung des Betrachtungsgerätes dieses relativ zu der Struktur so positioniert werden, daß der Durchstoßpunkt in der optischen Achse angeordnet wird, d.h. der Beobachter verschiebt das Betrachtungsgerät relativ zur Struktur so lange, bis die Markierung des Durchstoßpunktes mit dem Fadenkreuz in der Abbildung ubereinstimmt. Erfolgt dies in jeder Ebene, so ist. sichergestellt, daß das optische Betrachtungsgerät längs des vorbestimmten Verschiebeweges verschoben wird.

Ist der Verschiebeweg für ein Instrument bestimmt, so beobachtet der Benutzer die tatsächliche Position des Instrumentes in der beobachteten Ebene und verschiebt das Instrument seitlich so, daß es mit der Markierung des Durchstoßpunktes in Deckung kommt. Erfolgt dies in allen Ebenen, so ist sichergestellt, daß das Instrument längs des vorbestimmten Verschiebeweges geführt wird. Dabei ist wesentlich, daß eine solche Führung des Instrumentes längs des Verschiebeweges unabhängig davon ist, wie das Beobachtungsgerät relativ zu der Struktur genau angeordnet ist, da durch die dauernde Messung der Relativposition des Betrachtungsgerätes relativ zu der Struktur und durch Vergleich des so gewonnenen Datensatzes mit den Koordinaten des vorbestimmten Verschiebeweges auch eine entsprechende Änderung des Abstandes und der Richtung des markierten Punktes vom Fadenkreuz erfolgt, so daß immer die relative Lage des Durchstoßpunktes relativ zum momentanen Fokuspunkt des Betrachtungsgerätes angezeigt wird.

Bei dem beschriebenen Verfahren muß zur Korrektur der Verschiebebewegung eines Instrumentes oder des Betrachtungsgerätes eine Ebene nach der anderen beobachtet werden, wobei die Durchstoßpunkte entsprechend dem vorbestimmten Verschiebeweg wandern. Bei einer bevorzugten Ausführungsform ist vorgesehen, daß man in jeder dargestellten Ebene neben den Durchstoßpunkten des Verschiebeweges zusätzlich den oder die Durchstoßpunkte des Verschiebeweges mindestens einer der Betrachtungsebene benachbarten Ebene darstellt. Es werden also in der beobachteten Darstellung nicht nur die Durchstoßpunkte in der beobachteten Ebene dargestellt, sondern auch Durchstoßpunkte beispielsweise in einer darüber und einer darunter liegenden Parallelebene. Wenn der Verschiebeweg senkrecht zu den Ebenen verläuft, ergibt sich nach wie vor nur eine Markierung, wenn er jedoch gegenüber den beobachteten Ebenen geneigt ist, liegen die entsprechenden Markierungspunkte in der Darstellung nebeneinander. Die Betrachtungsperson kann daher auch erkennen, in welcher Richtung beispielsweise ein Instrument bewegt werden muß, wenn das Instrument senkrecht zur beobachteten Ebene in eine tieferliegende oder eine höherliegende Ebene verschoben wird. Dies kann für eine größere Anzahl von Ebenen der Fall sein, so daß in die beobachtete Ebene praktisch der Verschiebeweg projiziert wird.

Dabei ist es günstig, wenn die Durchstoßpunkte verschiedener Ebenen in der Darstellung durch eine Linie verbunden werden.

Weiterhin ist es vorteilhaft, wenn der oder die Durchstoßpunkte durch die beobachtete Ebene verschieden von Durchstoßpunkten in anderen Ebenen dargestellt sind. Dies erleichtert die Verschiebung des Instrumentes oder des Beobachtungsgerätes.

Bei dem vorstehend näher erläuterten Verfahren wird die vom Betrachtungsgerät abgebildete Ebene der Arbeitsstruktur zweidimensional abgebildet, dementsprechend werden Durchstoßpunkte des Verschiebeweges durch diese Ebene oder die Projektion des geplanten Verschiebeweges in diese Ebene dargestellt.

Bei einer abgewandelten Ausführungsform ist vorgesehen, daß man den Arbeitsbereich stereoskopisch betrachtet und dreidimensional abbildet und daß man den Verschiebeweg als dreidimensionale Darstellung der dreidimensionalen Abbildung des Arbeitsbereiches ortsrichtig überlagert. Dies kann beispielsweise durch an sich bekannte stereoskopische Betrachtung erfolgen, bei der zwei getrennte Abbildungen überlagert werden, die beim Betrachter einen dreidimensionalen Eindruck erwecken. In den Strahlengang kann in geeigneter Weise ein Bild eingelagert werden, das ebenfalls dreidimensional den gewünschten Verschiebeweg darstellt, der in der dreidimensionalen tatsächlichen Abbildung ortsrichtig gesehen wird. Dabei kann in ähnlicher Weise vorgegangen werden, wie dies beispielsweise von Anzeigeinstrumenten für Flugzeuge bekannt ist, mit denen auch im Blickfeld des Betrachters an einer bestimmten Position dreidimensional erscheinende Bilder erzeugt werden.

Dabei ist es vorteilhaft, wenn man den Verschiebeweg im Durchstoßpunkt der durch den Brennpunkt des Betrachtungsgerätes laufende Betrachtungsebene kennzeichnet, beispielsweise durch andere Farbgebung oder andere Helligkeit. Dies zeigt dem Betrachter die Lage der scharf abgebildeten Ebene des Arbeitsbereiches an, gleichzeitig kann er darüber und darunter liegende Bereiche des Arbeitsbereiches dreidimensional sehen und den Verlauf des Verschiebeweges in diesem Bereich.

Die Koordinaten des Verschiebeweges kann man beispielsweise mittels einer Vielzahl von Schnittebenendarstellungen der Struktur und in diesen festgelegten Durchstoßpunkten des Verschiebeweges bestimmen.

So können in herkömmlicher Weise mit Hilfe eines Röntgenstrahlungs- oder eines Kernspintomographen die entsprechenden Strukturen voruntersucht werden, d.h. es werden Schnittbilddarstellungen der Struktur angefertigt. In diesen Schnittbilddarstellungen oder in den entsprechenden Datensätzen werden die gewünschten Verschiebewege festgelegt, d.h. es werden in diesen Ebenen die Koordinaten der Durchstoßpunkie des Verschiebeweges bestimmt. Nimmt man diese Daten zusammen, so erhält man einen dreidimensionalen Datensatz, der den Verschiebeweg innerhalb der Struktur vom Anfang bis zum Ende beschreibt.

Dieser Datensatz wird in der beschriebenen Weise mit den Daten verglichen, die die jeweilige Positionierung des Betrachtungsgerätes relativ zu der Struktur und damit die Lage der beobachteten Ebene in der Struktur beschreiben.

Die genannte Aufgabe wird erfindungsgemäß weiterhin gelöst durch eine Vorrichtung zur Darstellung eines Arbeitsbereichs in einer dreidimensionalen Struktur mit einem Betrachtungsgerät, welches eine Ebene des Arbeitsbereichs scharf abbildet, mit einer Meßeinrichtung zur Bestimmung der Position des Betrachtungsgeräts relativ zu der Struktur, mit einer Vergleichseinrichtung zum Vergleich der für jede beobachtete Ebene geltenden Positionsdaten des Betrachtungsgeräts mit den Koordinaten eines vorbestimmten Verschiebewegs im beobachteten Arbeitsbereich, mit einer Darstellungseinheit und mit einem Datenspeicher, in dem ein Datensatz für den Verschiebeweg relativ zur Struktur gespeichert ist, wobei die Darstellungseinheit derart ausgebildet ist, daß sie ausgehend von diesem Datensatz den Durchstoßpunkt dieses Verschiebewegs in der jeweils beobachteten Ebene des Arbeitsbereichs ortsrichtig abbildet.

Die nachfolgende Beschreibung einer bevorzugten Ausführungsform der Erfindung dient im Zusammenhang mit der Zeichnung der näheren Erläuterung. Es zeigen:
- Fig. 1: eine schematische Darstellung eines Be-obachtungsgerätes mit einer Positionsmeßeinrichtung und einer Vorrichtung zur ortsrichtigen Abbildung des gewünschten Verschiebeweges und
- Fig. 2: eine Darstellung der für die Beobachtungsperson beobachtbaren Abbildung mit einer Darstellung des vorbestimmten Verschiebeweges und einer Darstellung eines Instrumentes.

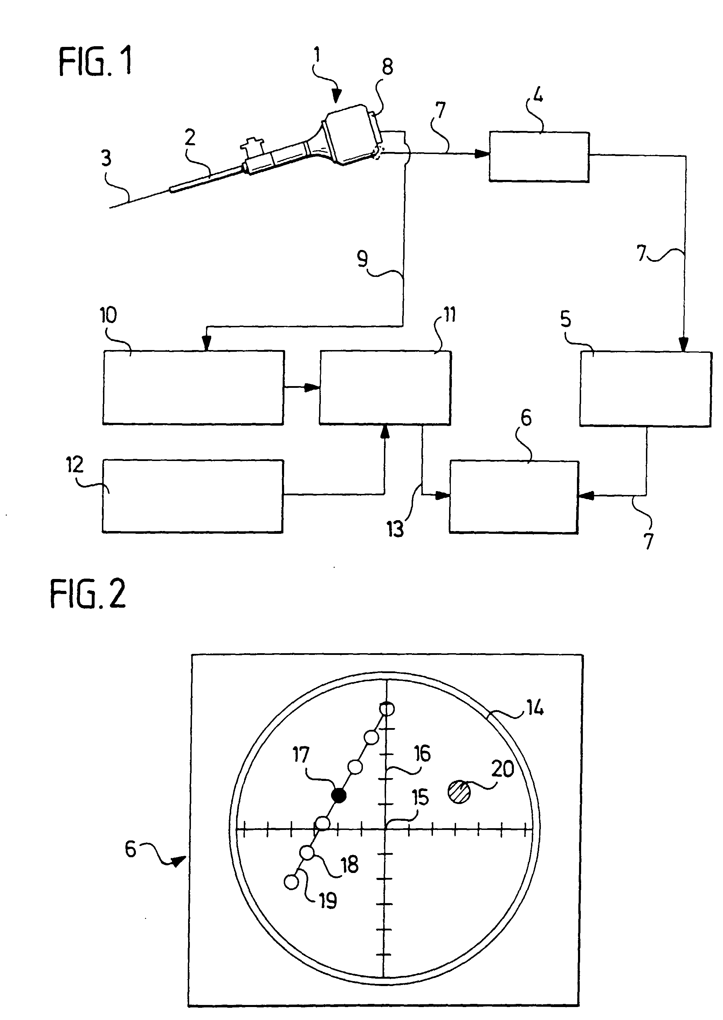

In der Darstellung der Fig. 1 wird ein Endoskop 1 verwendet, um eine zu untersuchende Struktur, die in der Zeichnung nicht dargestellt ist, zu betrachten. Das Endoskop 1 weist dazu in einem Rohr 2 eine nicht näher dargestellte Optik auf, die in einem bestimmten Abstand vor dem Ende des Rohres 2 eine Brennebene aufweist. Bei dem in der Figur dargestellten Ausführungsbeispiel ist an das Rohr 2 ein Instrument 3 in Form einer Spitze gehalten, welches in der Brennebene endet. Das Instrument 3 markiert also mit seinem vorderen Ende den Brennpunkt der Betrachtungsoptik und wird gleichzeitig als Tastinstrument, als Elektrode, als Sonde oder dergleichen, verwendet.

Das vom optischen System des Endoskops 1 übertragene Licht wird in einem Bildumsetzer 4 in elektrische Signale umgewandelt, die nach entsprechender Bildverarbeitung in einer Bildverarbeitungseinheit 5 über eine Leitung 7 einem Monitor 6 zugeführt werden, auf dem die mittels des Endoskops 1 betrachtete Fläche dargestellt wird.

In der Bildverarbeitungseinheit 5 kann das Bild in an sich bekannter Weise elektronisch verarbeitet werden, beispielsweise durch Konstrastverstärkung, durch spezielle Einfärbungstechniken oder durch Vergrößerungen etc.

Die exakte Positionierung des Endoskopes 1 relativ zu der zu betrachtenden Struktur wird über geeignete Sensoren 8 bestimmt, und die dabei erzeugten Positionssignale werden über eine Leitung 9 einem Positionsspeicher 10 zugeführt. Die Sensoren 8 können beispielsweise Ultraschallsender sein, die mit entsprechenden Ultraschallmikrophonen an der Struktur zusammenwirken, so daß durch verschiedene Laufzeitmessungen die Relativpositionierung feststellbar ist.

Die die jeweilige Positionierung bestimmenden Signale werden aus dem Positionsspeicher 10 einer Vergleichseinrichtung 11 zugeführt, die außerdem von einem Datenspeicher 12 aus mit Datensätzen versorgt wird, durch die die Koordinaten des gewünschten Verschiebeweges des Endoskopes oder eines Instrumentes in der zu beobachtenden Struktur beschrieben werden.

Diese Datensätze werden durch vorherige Strukturbestimmungen und durch Vorgabe des gewünschten Verschiebeweges in der so bestimmten Struktur gewonnen.

In der Vergleichseinrichtung 11 werden die Positonsdaten des Endoskops 1 mit diesen Datensätzen des gewünschten Verschiebeweges verglichen, so daß genau festgestellt werden kann, an welcher Stelle der betrachteten Ebene der Verschiebeweg diese Ebene durchsetzt. Ebenso können in der Vergleichseinrichtung 11 entsprechende Daten für die Ebenen bestimmt werden, die über bzw. unter der betrachteten Ebene liegen, so daß für die verschiedenen Durchstoßpunkte des Verschiebeweges und dieser Ebenen entsprechende Koordinaten zur Verfügung stehen. Diese Koordinaten werden über eine Leitung 13 auf den Monitor 6 übertragen und führen dort zur Markierung der entsprechenden Koordinaten des Durchstoßpunktes in der dargestellten Ebene, d.h. es wird ein Bild einer solchen Markierung mit der unmittelbar vom Endoskop 1 gewonnenen Abbildung erzeugt.

In Fig. 2 ist ein mögliches Bild einer Monitordarstellung gezeigt. Der gesamte, durch das Endoskop 1 erreichbare Sichtbereich ist in einen Kreis 14 eingefaßt, dessen Mittelpunkt 15 durch ein Fadenkreuz 16 markiert ist.

Der Mittelpunkt 15 fällt mit der optischen Achse des Endoskops 1 zusammen, so daß innerhalb des Kreises 14 eine Abbildung der Struktur in der Ebene erzeugt wird, die senkrecht auf der optischen Achse steht und mit der Brennebene des Endoskops 1 zusammenfält. Dabei markiert der Mittelpunkt 15 den Durchstoßpunkt der optischen Achse durch diese Ebene.

Außerdem erkennt man in dem durch den Kreis 14 umschriebenen Bereich mehrere zusätzliche Markierungen, nämlich einen ausgefüllten Punkt 17 und mehrere kreisförmige Punkte 18, die alle untereinander durch eine Linie 19 verbunden sind.

Der ausgefüllte Punkt 17 markiert den Durchstoßpunkt der vorbestimmten und gewünschten Verschiebelinie durch die betrachtete Ebene, die Kreise 18 auf der einen Seite des Punktes 17 entsprechende Durchstoßpunkte in oberhalb der betrachteten Ebene liegenden Parallelebenen und die Kreise auf der anderen Seite des Punktes 17 entsprechende Durchstoßpunkte in unterhalb der betrachteten Ebene liegenden Parallelebenen. Durch die Kreise 18 und den Punkt 17 wird also eine Projektion des Verschiebeweges auf die betrachtete Ebene dargestellt, wobei der Durchstoßpunkt des Verschiebeweges durch den Punkt 17 markiert wird.

Die Lage, in der die Kreise und Punkte auf dem Monitor dargestellt werden, ergibt sich aus dem Datensatz des vorbestimmten Verschiebeweges. Durch die Positionsmessung des Endoskopes 1 laßt sich die Lage der beobachteten Ebene mathematisch beschreiben, so daß man durch ein mathematisches Schneiden des Verschiebeweges und dieser Ebene den Durchstoßpunkt errechnen kann, d.h. den Abstand von der optischen Achse und den Winkel gegenüber einer bestimmten Richtung. Diese Daten reichen aus, um den Durchstoßpunkt auf dem Monitor abzubilden.

Wenn der Benutzer beispielsweise das Instrument 3 längs des vorbestimmten Verschiebeweges führen will, erkennt er aus der Darstellung der Fig. 2, daß die Spitze des Instrumentes, die mit der optischen Achse zusammenfällt und damit durch das Fadenkreuz 16 markiert wird, von dem Durchstoßpunkt des gewünschten Verschiebeweges entfernt ist. Der Benutzer kann durch Verschiebung des Endoskopes und des daran gehaltenen Instrumentes erreichen, daß der Punkt 17 auf dem Monitorbild in das Fadenkreuz verschoben wird. Wird dies erreicht, so ist sichergestellt, daß die Spitze des Instrumentes die beobachtete Ebene genau im Durchstoßpunkt des Verschiebeweges durchsetzt, also in der gewünschten Weise positioniert ist. Schiebt man das Endoskop tiefer in die Struktur ein, erreicht man darunterliegende Ebenen, die dann dargestellt werden, wobei die Kreise 18 dem Benutzer bereits vor dem Verschieben des Endoskops in eine tiefere Lage angeben, in welcher Richtung des Endoskops seitlich zu verschieben ist, um längs des Verschiebeweges weitergeführt zu werden. Der Benutzer kann also beim Einschieben des Endoskops die Spitze des Instrumentes 3 längs des Verschiebeweges bewegen, wenn er sich bemüht, den jeweiligen Punkt 17, der selbstverständlich für jede beachtete Ebene verschieden ist, immer im Fadenkreuz zu halten.

Bei einem anderen Ausführungsbeispiel, bei dem ein separates Instrument benutzt wird, wird der Durchstoßpunkt dieses Instrumentes durch die beobachtete Ebene auf dem Monitor beobachtbar. Dieser Durchstoßpunkt ist in der Darstellung der Fig. 2 mit dem Bezugszeichen 20 gekennzeichnet.

Um sicherzustellen, daß dieses Instrument immer längs des gewünschten Verschiebeweges verschoben wird, muß das Instrument so lange seitlich verschoben werden, bis der Durchstoßpunkt mit dem markierten Punkt 17 in der Darstellung zur Deckung gelangt. Damit ist gewährleistet, daß der Durchstoßpunkt des Instrumentes durch die beobachtete Ebene auf dem gewünschten Verschiebeweg liegt. Allerdings ist damit noch nicht gewährleistet, daß auch die Einschubtiefe des Instrumentes stimmt, hierzu müßten gegebenenfalls zusätzliche Maßnahmen gefunden werden, beispielsweise eine bestimmte Ausbildung des Instrumentes nur in seinem Endbereich, so daß diese bestimmte Formgebung in der beobachteten Ebene überwacht werden kann, da sich das Ende des Instrumentes gerade in der jeweils beobachteten Ebene befindet.

Bei dieser Art der Verwendung muß das Endoskop nicht unbedingt dem Verschiebeweg folgen, sondern es kann auch einen anderen Weg einschlagen, da durch die jeweilige Positionsmessung des Endoskops und durch den Vergleich der Datensätze gewährleistet ist, daß bei jeder Positionierung des Endoskops in der jeweils betrachteten Ebene der Durchstoßpunkt des Verschiebeweges an der örtlich richtigen Stelle angezeigt wird.

Verwendet man die beschriebene Vorrichtung beispielsweise bei der Operation an einem Menschen, so wird zunächst vor der Operation bei noch nicht geöffnetem Körper durch konventionelle Techniken, beispielsweise durch übliche Computertomographie oder durch Kernspintomographie durch eine Vielzahl von Schnittbildern die dreidimensionale Formgebung des Körpers bestimmt, also die Struktur. In die entsprechenden Schnittbilder kann in jede Ebene der gewünschte Verschiebeweg, d.h. der jeweilige Durchstoßpunkt des Verschiebeweges, eingefügt werden, so daß man auf diese Weise durch Interpolation einerseits einen Datensatz für die Beschreibung der dreidimensionalen Struktur und andererseits einen weiteren Datensatz für die Koordinaten des Verschiebeweges in dieser Struktur erhält.

Bei der eigentlichen Operation wird das Endoskop und gegebenenfalls ein zusätzliches Instrument durch Körperöffnungen in den Körper eingeführt. Auf dem Monitor wird jeweils die in der Brennebene des Endoskops liegende Ebene der Struktur abgebildet. Außerdem erkennt man auf dem Monitor den Durchstoßpunkt des vorbestimmten Verschiebeweges durch die beobachtete Ebene, d.h. der Operateur kann beispielsweise durch seitliche Verschiebung des Endoskopes gewährleisten, daß dieses genau auf dem vorbestimmten Verschiebeweg in die Struktur eingeführt wird. Diese Korrektur kann anhand des Punktes 17 in jeder beliebigen Ebene erfolgen, die Kreise 18 zeigen dem Operateur weiter, in welcher Richtung des Endoskops beim weiteren Eintauchen zu verschieben ist, um auch in darunterliegenden Ebenenen längs des gewünschten Verschiebeweges zu bleiben.

Vorstehend ist die Erfindung anhand einer zweidimensionalen Darstellung des Arbeitsbereiches beschrieben worden, bei der also jeweils eine in der Brennebene des Betrachtungsgerätes liegende Ebene des Arbeitsbereiches scharf abgebildet wird. Bei einem in der Zeichnung nicht eigens dargestellten Ausführungsbeispiel laßt sich dies so abwandeln, daß beispielsweise mittels einer stereoskopischen Betrachtung durch Überlagerung von zwei geringfügig unterschiedlichen Teilbildern eine dreidimensional erscheinende Abbildung des Arbeitsbereiches erzeugt wird. Dieser dreidimensional erscheinenden Abbildung wird dann der vorbestimmte Verschiebeweg ebenfalls dreidimensional überlagert, so daß der Betrachter den Verlauf des Verschiebeweges im Arbeitsbereich über eine bestimmte Schichtdicke desselben beobachten kann. Vorteilhafterweise wird dabei der Durchstoßpunkt des Verschiebeweges durch die in der Brennebene des Betrachtungsgerätes liegende Ebene markiert, sei es durch eine abweichende Farbe, eine abweichende Helligkeit oder eine unterschiedliche Form, z.B. eine Verdickung. Grundsätzlich ist bei dieser Ausführung der Erfindung jedoch in gleicher Weise wesentlich, daß die Positionierung des Betrachtungsgerätes gegenüber der dreidimensionalen Struktur laufend gemessen und in Abhängigkeit von dieser Messung die in einem Speicher abgelegten Daten des Verschiebeweges ortsrichtig in die Darstellung übertragen werden.

## Patentansprüche

1. Verfahren zur Darstellung eines Arbeitsbereiches in einer dreidimensionalen Struktur, bei dem man den Arbeitsbereich mit einem Betrachtungsgerät abbildet, die Positionierung des Betrachtungsgerätes relativ zu der Struktur bestimmt, die Koordinaten eines Verschiebeweges für ein Instrument oder für das Betrachtungsgerät in der Struktur vorbestimmt, sie mit der jeweiligen Positionierung vergleicht, dadurch gekennzeichnet, daß man in der Abbildung des Arbeitsbereiches den vorbestimmten Verschiebeweg ortsrichtig als Durchstoßpunkt des Verschiebewegs durch die jeweils beobachtete Ebene der Struktur darstellt.

2. Verfahren nach Anspruch 1, dadurch gekennzeichnet, daß man in jeder dargestellten Ebene neben den Durchstoßpunkten des Verschiebeweges zusätzlich den oder die Durchstoßpunkte des Verschiebeweges mindestens einer der Betrachtungsebene benachbarten Ebene darstellt.

3. Verfahren nach Anspruch 2, dadurch gekennzeichnet, daß die Durchstoßpunkte verschiedener Ebenen in der Darstellung durch eine Linie verbunden werden.

4. Verfahren nach einem der Ansprüche 2 oder 3, dadurch gekennzeichnet, daß der oder die Durchstoßpunkte durch die beobachtete Ebene verschieden von Durchstoßpunkten in anderen Ebenen dargestellt sind.

5. Verfahren nach Anspruch 1, dadurch gekennzeichnet, daß man den Arbeitsbereich dreidimensional abbildet und in bekannter Weise stereoskopisch betrachtet und daß man den Verschiebeweg als dreidimensionale Darstellung der dreidimensionalen Abbildung des Arbeitsbereiches ortsrichtig überlagert.

6. Verfahren nach Anspruch 5, dadurch gekennzeichnet, daß man den Verschiebeweg im Durchstoßpunkt der durch den Brennpunkt des Betrachtungsgerätes laufenden Beobachtungsebene kennzeichnet.

7. Verfahren nach einem der voranstehenden Ansprüche, dadurch gekennzeichnet, daß man die Koordinaten des Verschiebeweges mittels einer Vielzahl von Schnittebenendarstellungen der Struktur und in diesen festgelegten Durchstoßpunkten des Verschiebeweges bestimmt.

8. Vorrichtung zur Darstellung eines Arbeitsbereiches in einer dreidimensionalen Struktur mit einem Betrachtungsgerät (1), welches eine Ebene des Arbeitsbereiches scharf abbildet, mit einer Meßeinrichtung (8, 10) zur Bestimmung der Position des Betrachtungsgerätes (1) relativ zu der Struktur, mit einer Vergleichseinrichtung zum Vergleich der für jede beobachtete Ebene geltenden Positionsdaten des Betrachtungsgerätes (1) mit den Koordinaten eines vorbestimmten Verschiebeweges im beobachteten Arbeitsbereich, mit einer Darstellungseinheit (6) und mit einem Datenspeicher (12), in dem ein Datensatz für den Verschiebeweg relativ zur Struktur gespeichert ist, wobei die Darstellungseinheit (6) derart ausgebildet ist, daß sie ausgehend von diesem Datensatz den Durchstoßpunkt (17) dieses Verschiebeweges in der jeweils beobachteten Ebene des Arbeitsbereichs ortsrichtig abbildet.

## Claims

1. A method of representing a work area in a three-dimensional structure, wherein the work area is projected by a viewing device, the position of the viewing device relative to the structure is determined, the co-ordinates of a displacement path for an instrument or for the viewing device in the structure are predetermined and compared with the actual position, characterised in that, in the projection of the work area, the predetermined displacement path is locally accurately represented as a piercing point of the displacement path through the observed plane of the structure.

2. A method according to claim 1, characterised in that, in each plane represented, apart from the piercing points of the displacement path, the piercing point or points of the displacement path in at least one plane adjacent to the viewing plane are additionally represented.

3. A method according to claim 2, characterised in that the piercing points of different planes are connected by a line in the representation.

4. A method according to one of claims 2 and 3, characterised in that the piercing point or points through the observed plane are represented differently from piercing points in other planes.

5. A method according to claim 1, characterised in that the work area is projected three-dimensionally and viewed stereoscopically in a known manner, and in that the displacement path is locally accurately superimposed as a three-dimensional representation on the three-dimensional projection of the work area.

6. A method according to claim 5, characterised in that the displacement path is identified by the piercing point in the observation plane extending through the focal point of the viewing device.

7. A method according to any one of the preceding claims, characterised in that the co-ordinates of the displacement path are determined by means of a plurality of cutting-plane representations of the structure and piercing points of the displacement path established in the said representations.

8. A device for representing a work area in a three-dimensional structure by means of a viewing device (1) sharply projecting a plane of the work area, comprising a measuring device (8, 10) for determining the position of the viewing device (1) relative to the structure, a comparator for comparing the positional data from the viewing device (1) for each plane observed with the co-ordinates of a predetermined displacement path in the work area observed, a display unit (6) and a data store (12), in which a data set for the displacement path relative to the structure is stored, wherein the display unit (6) is formed in such a manner that, on the basis of this data set, it locally accurately projects the piercing point (17) of this displacement path in the observed plane of the work area.

## Revendications

1. Procédé de représentation d'une zone de travail d'une structure tridimensionnelle, dans le cas duquel un appareil d'observation donne une image de la zone de travail, on détermine la position de l'appareil d'observation par rapport à la structure, on prédétermine les coordonnées d'un chemin de translation pour un instrument ou pour l'appareil d'observation dans la structure, on les compare avec la position respective, caractérisé par le fait que, sur l'image, ainsi obtenue, de la zone de travail, on représente en position correcte le chemin de translation prédéterminé sous forme du point où le chemin de translation traverse le plan de la structure chaque fois observé.

2. Procédé selon la revendication 1, caractérisé par le fait que, dans chaque plan représenté, à côté des points de traversée du chemin de translation, on présente en outre le ou les points où le chemin de translation traverse au moins l'un des plans d'observation voisins.

3. Procédé selon la revendication 2, caractérisé par le fait que, les points de traversée à travers les différentes plans sont reliés dans la représentation par une droite.

4. Procédé selon l'une des revendications 2 ou 3, caractérisé par le fait que le ou les points de traversée à travers le plan observé sont représentés de façon différente des points de traversée à travers d'autres plans.

5. Procédé selon la revendication 1, caractérisé par le fait que l'on obtient une image tridimensionnelle de la zone de travail et que, de façon connue, on l'observe en mode stéréoscopique et que l'on superpose en position correcte le chemin de translation sous forme d'une représentation tridimensionnelle de l'image tridimensionnelle de la zone de travail.

6. Procédé selon la revendication 5, caractérisé par le fait que l'on caractérise le chemin de translation au point de traversée du plan d'observation passant par le point focal de l'appareil d'observation.

7. Procédé selon l'une des revendications précédentes, caractérisé par le fait que l'on détermine les coordonnées du chemin de translation au moyen d'une pluralité de représentations de plans de coupe de la structure et de points de traversée du chemin de translation qui y sont définis.

8. Dispositif de représentation d'une zone de travail d'une structure tridimensionnelle comportant un appareil d'observation (1) qui donne une image nette d'un plan de la zone de travail, un instrument de mesure (8, 10) pour déterminer la position de l'appareil d'observation (1) par rapport à la structure, un instrument de comparaison pour comparer les données de position, valant pour chaque plan observé, de l'appareil d'observation (1) avec les coordonnées d'un chemin de translation prédéterminé dans la zone de travail observée, un organe de représentation (6) et une mémoire de données (12) dans laquelle est mémorisé un enregistrement de données pour le chemin de translation par rapport à la structure, l'organe de représentation (6) étant conçu de façon qu'en partant de cet enregistrement de données, il donne, en position correcte, une image du point de traversée (17) de ce chemin de translation dans le plan de la zone de travail chaque fois observé.
